(19) Europäisches Patentamt
European Patent Office
Office européen des brevets

(11) EP 4 386 768 A1

(12) EUROPEAN PATENT APPLICATION

(43) Date of publication:
19.06.2024  Bulletin 2024/25

(51) International Patent Classification (IPC):
*G16H 10/60* (2018.01)  *G16H 20/70* (2018.01)

(21) Application number: 23154488.3

(22) Date of filing: 01.02.2023

(52) Cooperative Patent Classification (CPC):
G16H 10/60; G16H 20/70

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 13.12.2022 US 202263432102 P

(71) Applicant: Koninklijke Philips N.V.
5656 AG Eindhoven (NL)

(72) Inventors:
• PAUWS, Steffen Clarence
5656AG Eindhoven (NL)
• WIRTH, Christoph Tobias
5656AG Eindhoven (NL)
• SOKORELI, Ioanna
5656AG Eindhoven (NL)

(74) Representative: Philips Intellectual Property &
Standards
High Tech Campus 52
5656 AG Eindhoven (NL)

(54) **ACTIVE SLEEP APNEA CASE FINDING AND SCHEDULING SYSTEM AND METHOD OF OPERATION THEREOF**

(57) A screening system (100) including a controller (120, 190, 194) to: query data sources (142, 144, 146, 148, 150, 152) to extract medical status information (MSI) of a patient cohort; form patient cohort data (PCD) comprising records of the extracted MSI of the patients; exclude records that are disqualified according to exclusion rules (ERS), and update the PCD accordingly; determine risk factors that are present for each record in accordance with a comparison of the MSI for corresponding records with risk factors for obstructive sleep apnea (OSA); determine a total risk score for each in accordance with a total of the determined risk factors present; determine a predicted probability of OSA based upon the determined total risk score for each of the records; assign each to a risk category in accordance with risk thresholds applied to the predicted probability of OSA; and determine a follow-up procedure for each in accordance with the assigned risk category for the records.

FIG. 1

EP 4 386 768 A1

**Description**

FIELD OF THE INVENTION

The present system relates to a system that screens individuals for suspected obstructive sleep apnea and, more particularly, to a system that utilizes health information to screen individuals for suspected obstructive sleep apnea to enable active case-finding, diagnosis and treatment and a method of operation thereof.

BACKGROUND OF THE INVENTION

Undiagnosed health conditions are a major problem faced by the general population. Oftentimes, these undiagnosed health conditions persist until a significant event occurs that is obviously manifested, thereby enabling identification of at least the manifestation (e.g., heart attack) and follow-on testing to identify root causes of the manifestation. For example, undiagnosed obstructive sleep apnea (OSA) is a prevalent and worrying health issue for many individuals. The prevalence of obstructive sleep apnea (that is, its diagnosis) associated with accompanying daytime sleepiness is approximately 3 to 7% for adult men and 2 to 5% for adult women in the general population. The prevalence varies in subpopulations, being higher, for instance, with overweight and obese cohorts or older individuals (e.g., see, URL "ncbi.nlm.nih.gov/pmc/articles/PMC2645248/"). A Lancet review publication, entitled "Estimation of the Global Prevalence and Burden of Obstructive Sleep Apnea: A Literature-Based Analysis", by Adam B. Benjafield, et. al., reported almost one billion people are affected with OSA worldwide, (e.g., see, URL "thelancet.com/journals/lanres/article/PIIS2213-2600(19)30198-5/fulltext"). Recognition of this problem calls for effective diagnostic and treatment strategies to curb the disease burden and reduce the medical cost associated therewith.

There is an enormous under-diagnosis of OSA further rising public health costs and individual burden. The prevalence of under-diagnosis of OSA is extremely high, and is believed above 50%, for almost all countries. More than 25 million adults in the United States are suspected to have moderate to severe OSA which is about equally prevalent as diabetes and even more prevalent than asthma. Individuals are unaware of risk factors and usually do not recognize or underestimate the first symptoms (e.g., disturbed sleep, daytime sleepiness, fatigue, irritability, and memory problems) of OSA, if these symptoms are present at all. Similarly, general practitioners (GPs) are not equally well-trained on assessing patients on OSA-related signs and symptoms. If OSA is not recognized and thus left untreated in individuals, OSA can have a negative impact on an affected individual's everyday quality-of-life, mood, productivity, and safety in daily life due to daytime sleepiness. Since the indications of OSA are often ignored until there is some overt manifestation, there is a significant increase in the costs associated with treating OSA following the overt manifestation. For example, OSA increases cardiovascular risk including risks of systemic hypertension, pulmonary hypertension, all-cause mortality, congestive heart failure, cardiovascular disease, atrial fibrillation, stroke, arrhythmias, and myocardial infarction (MI), all of which can increase healthcare utilization costs of these individuals once these symptoms are manifested or otherwise identified. In addition, OSA may result in fatigue, episodes of drowsy driving, increased risk of accidents and other quality of life problems or symptoms, such as a decrease in daytime vitality.

A diagnosis of OSA typically requires a sleep study such as full polysomnography, which is an expensive and time-consuming procedure that is performed in a sleep clinic. Therefore, sending an individual unnecessarily to a sleep clinic for a diagnosis should be avoided. Further, polysomnography is not readily available for many individuals with suspected OSA, mainly due to its cost ( e.g., which may be thousands of dollars) but also the long waiting lines for people to undergo the test. Less expensive and more accessible procedures such as home-based nasal airflow analysis or a home sleep study can be used to aid the diagnosis, or pre-diagnosis of OSA but these are less accurate and may often misdiagnose OSA. Thus, accurately recognizing latent OSA in individuals enabling treatment is vital to reduce disease burden and improve quality-of-life in the general population.

As discussed above, although OSA is quite prevalent among the mass population, screening an asymptomatic mass population on obstructive sleep apnea (OSA) is not currently costeffective and thus not recommended. Although cases of OSA will be found, mass screening is expected to have limited impact on health outcomes but will require an undue amount of effort and cost. In addition, the response rate (e.g., participation) of a mass screening instrument can be rather disappointing, resulting in self-selection and bias in the data and the screening results.

Typically, a diagnosis of OSA occurs when signs and symptoms are developed and present. However, OSA can start before signs and symptoms develop or can be recognized by patients, their partners, or physicians, which is known as early onset of OSA. In principle, the prevalence for OSA among the general population is considered sufficiently high to justify the use of a screening instrument, if the screening method is good enough (i.e., has high test validity) and cost effective. Early recognition of OSA can initiate effective treatment.

In order to reduce expensive and cumbersome diagnostic polysomnography on negative cases (i.e., patients without OSA), various clinical screening tools for sleep apnea have been created that are used in clinics as a patient survey which are known as: Stop, STOP-BANG™ (SB), Epworth Sleepiness Scale™ (ESS), 4-Variable screening tool™

(4-V) and Berlin Questionnaire™. These clinical screening tools vary in purpose (e.g., estimating high risk of OSA, detecting daytime sleepiness or insomnia), ease of administration and level of sensitivity versus specificity. Most screening tools have four to eight items and are reasonably easy to complete. Others have many more items to complete and are more complex. An ideal screening test for OSA should have high validity in testing, that is, being extremely sensitive and extremely specific. STOP-BANG™ is considered by some to be the most sensitive clinical screening tool to detect unrecognized sleep apnea. However, these clinical screening tools are hampered by low levels of specificity and do not perform sufficiently well in correctly identifying individuals who do not happen to have OSA (e.g., result in a high number of false positive assessments). Especially in primary care and screening, highly specific tests are required to reduce false positives, just to contain the cost in follow-up diagnosis and reduce patient concerns about a positive test result which turns out to be false, which can greatly inconvenience the individual who was misdiagnosed at great cost.

Accordingly, embodiments of the present system provide features and advantages which obviate conventional diagnostic systems and methods. To overcome the aforementioned barriers and detriments as well as others, there is a need for a system for mass screening a patient cohort for previously undiagnosed medical conditions among whom there may be a potential absence of clear symptoms in early onset of the medical condition such as provided by embodiments of the present system. The inefficiency of existing mass screening, the potential absence of clear symptoms in early onset of the medical condition, and low test validity of existing screening tools are a real problem and disadvantage to achieve high yield in finding, for example, unrecognized/undiagnosed OSA cases. Embodiments of the present system overcome these and other disadvantages of existing screening tools and methods and provide a system and method which can achieve high yield in finding patients that suffer from unrecognized/undiagnosed medical conditions which can justify expensive and time-consuming follow-on diagnostic testing to diagnose cases among the general population. It should also be appreciated that embodiments of the present system provide an efficient mass screening system and method for detecting early onset of given medical conditions with higher test validity than existing screening tools.

SUMMARY OF THE INVENTION

The system(s), device(s), method(s), arrangements(s), interface(s), computer program(s), processes, etc., (hereinafter each of which will be referred to as system, unless the context indicates otherwise), described herein address problems in prior art systems.

In accordance with embodiments of the present system, there is disclosed a_screening system including: at least one controller configured to: query a plurality of selected data sources for medical records to extract medical status information (MSI) of patients in a patient cohort; form patient cohort data (PCD) including patient case records, each of the patient case records including the extracted MSI of the patients in the patient cohort; exclude patient case records from the PCD that are determined to be disqualified according to exclusion rules (ERS), and update the PCD accordingly; determine risk factors that are present for each patient case record of the updated PCD in accordance with a comparison of the MSI for corresponding patient case records with risk factors for obstructive sleep apnea (OSA); determine a total risk score for each of the corresponding patient case records in accordance with a total of the determined risk factors that are determined to be present for the corresponding patient case records; determine a predicted probability of suspected OSA based upon a logistic function of the determined total risk score for each of the corresponding patient case records; assign each of the corresponding patient case records to a risk category in accordance with risk thresholds applied to the predicted probability of suspected OSA; and determine a follow-up procedure for each of the corresponding patient case records in accordance with the assigned risk category for the corresponding patient case records. The at least one controller may be further configured to render the determined follow-up procedure on a rendering device of the system under the control of the at least one controller. The at least one controller may be further configured to render one or more of the total risk score, the predicted probability of suspected OSA, and the determined risk category on the rendering device.

In accordance with embodiments, the at least one controller may be further configured to determine an estimated risk as at least one of odds, log odds, and probability based upon a logistic function of the determined total risk score for each of the corresponding patient case records. The at least one controller may be further configured to determine a system capacity limit. The at least one controller may be further configured to communicate with at least one sleep monitoring unit (SMU) (102) and to determine a cumulative capacity of the at least one SMU to conduct sleep tests and base the system capacity limit in accordance with a sum of the determined cumulative capacity. The at least one controller may be further configured to render a diagnostic follow-up based on the assigned category risk.

In addition, in accordance with embodiments of the present system, there is disclosed a screening system for an undiagnosed medical condition, including: at least one controller which is configured to: query a plurality of selected data sources for medical records to extract medical status information (MSI) of patients in a patient cohort; form patient cohort data (PCD) including patient case records, each of the patient case records including the extracted MSI of the patients in the patient cohort; exclude patient case records from the PCD that are determined to be disqualified according to exclusion rules (ERS), and update the PCD accordingly; determine risk factors that are present for each patient case record of the updated PCD in accordance with a comparison of the MSI for the corresponding patient case records with risk factors associated with the undiagnosed medical condition; determine a total risk score for each of the corresponding patient case records in accordance with a total of the determined risk factors that are determined to be present for the corresponding patient case records; determine a predicted probability of the undiagnosed medical condition based upon a logistic function of the determined total risk score for each of the corresponding patient case records; and assign each patient case record to a risk category or risk level in accordance with risk thresholds applied to the predicted probability of the undiagnosed medical condition. The at least one controller may be further configured to determine a follow-up procedure for each patient case record in accordance with the assigned risk category or risk level for the corresponding patient case records. The at least one controller may be further configured to render the determined follow-up procedure on a rendering device.

In accordance with embodiments, the at least one controller may be further configured to render one or more of the total risk score, the predicted probability of the undiagnosed medical condition, and the determined risk category or risk level on the rendering device. The at least one controller may be further configured to determine an estimated risk as at least one of odds, log odds, and probability based upon a logistic function of the determined total risk score for each of the corresponding patient case records. The at least one controller may be further configured to determine a system capacity limit. The at least one controller may be further configured to communicate with at least one monitoring unit and to determine a cumulative capacity of the at least one monitoring unit to conduct tests and base the system capacity limit in accordance with a sum of the determined cumulative capacity. The at least one controller may be further configured to render a diagnostic follow-up based on the assigned risk category or risk level.

Further, in accordance with embodiments of the present system, there is disclosed a method for screening for a given undiagnosed medical condition in a patient cohort, the method including acts of: querying a plurality of selected data sources for medical records to extract medical status information (MSI) of patients in the patient cohort; forming patient cohort data (PCD) including patient case records, each of the patient case records including the extracted MSI of the patients in the patient cohort; excluding patient case records from the PCD that are determined to be disqualified according to exclusion rules (ERS), and updating the PCD accordingly; determining risk factors that are present for each patient case record of the updated PCD in accordance with a comparison of the MSI for the corresponding patient case records with risk factors associated with the undiagnosed medical condition; determine a total risk score for each of the corresponding patient case records in accordance with a total of the determined risk factors that are determined to be present for the corresponding patient case records; determining a predicted probability of the undiagnosed medical condition based upon a logistic function of the determined total risk score for each of the corresponding patient case records; and assigning each patient case record to a risk category or risk level in accordance with risk thresholds applied to the predicted probability of the undiagnosed medical condition. The method may include an act of determining a follow-up procedure for each patient case record in accordance with the assigned risk category or risk level for the corresponding patient case records.

The method may include an act of rendering the determined follow-up procedure on a rendering device. The method may include acts of: annotating the updated PCD with one or more of the total risk score, the estimated risk predicted probability of the undiagnosed medical condition, and the determined risk category or risk level; and storing the annotated PCD on a storage device. The method may include acts of: determining a cumulative capacity of a plurality of monitoring units (102) to conduct tests; and determining a follow-up procedure for each patient case record in accordance with the assigned risk category or risk level for the corresponding patient case records and in accordance with the determined cumulative capacity of the plurality of monitoring units.

BRIEF DESCRIPTION OF THE DRAWINGS

It should be expressly understood that the drawings are included for illustrative purposes and do not represent the scope of the present system. It is to be understood that the figures may not be drawn to scale. Further, the relation between objects in a figure may not be to scale and may in fact have a reverse relationship as to size. The figures are intended to bring understanding and clarity to the structure of each object shown, and thus, some features may be exaggerated in order to illustrate a specific feature of a structure. In the accompanying drawings, like reference numbers in different drawings may designate identical or similar elements, portions of similar elements and/or elements with similar functionality. The present system is explained in further detail, and by way of example, with reference to the accompanying drawings which show features of various exemplary embodiments that may be combinable and/or severable wherein:

FIG. 1 illustratively shows a schematic block diagram of a portion of a system operating in accordance with embodiments of the present system;
FIG. 2 illustratively shows a functional flow diagram performed by a process in accordance with embodiments of the present system;

FIG. 3 illustratively shows OSA screening probability as a logistic function of the number of risk factor present in a patient - and contained in a OSA risk score in accordance with embodiments of the present system;

FIG. 4 illustratively shows the odds of being susceptible of having OSA as a function of the number of risk factor present in a patient - and contained in a OSA risk score in accordance with embodiments of the present system; and

FIG. 5 illustratively shows the odds of being susceptible of having OSA as a function of a scaled OSA score in accordance with embodiments of the present system.

DETAILED DESCRIPTION OF EMBODIMENTS

The following are descriptions of illustrative embodiments that when taken in conjunction with the following drawings will demonstrate the above noted features and advantages, as well as further ones. In the following description, for purposes of explanation rather than limitation, illustrative details are set forth such as architecture, interfaces, techniques, element attributes, etc. However, it will be apparent to those of ordinary skill in the art that other embodiments that depart from these details would still be understood to be within the scope of the appended claims. Moreover, for the purpose of clarity, detailed descriptions of well-known devices, circuits, tools, techniques, and methods are omitted so as not to obscure the description of the present system.

The term "and/or," and formatives thereof, should be understood to mean that only one or more of the recited elements may need to be suitably present (e.g., only one recited element is present, two of the recited elements may be present, etc., up to all of the recited elements may be present) in a system in accordance with the claims recitation and in accordance with one or more embodiments of the present system. In the context of the present embodiments, the terms "about", substantially and "approximately" denote an interval of accuracy that a person skilled in the art will understand to still ensure the technical effect of the feature in question which in some cases may also denote "within engineering tolerances." For example, the terms may indicate a deviation from the indicated numerical value(s) of $\pm 20$ %, $\pm 15$ %, $\pm 10$ %, $\pm 5$ %, $\pm 1$ % $\pm 0.5$ % or $\pm 0.1$ %.

The terms clinician, clinicians, and/or formatives thereof, may include a professional such as a doctor, a clinician, a technologist, an operator, an expert, medical specialist, medical specialist a technician, and/or the like, who may operate and/or review information related to the patient such as system information, and/or data generated by embodiments of the present system and associated data and/or the like unless the context indicates otherwise.

The terms patient, patients, and/or formatives thereof, may include patients or other individuals (e.g., persons, subjects, subjects-under-test, etc.) or other users who may be receiving any type of sleep apnea monitoring or assessment using systems, machines, processes, and/or methods operating in accordance with embodiments of the present system.

The present system relates to a system and method to provide for pre-screening of individuals for given suspected undiagnosed medical condition(s). To simplify the following discussion, obstructive sleep apnea (OSA) is discussed as an illustrative medical condition that may be identified in accordance with embodiments of the present system. However, the discussion regarding OSA herein, should be understood to apply to other medical conditions with suitable adjustments to system portions and as such, are understood to be within the scope of the appended claims unless explicitly indicated otherwise. For example, the present system may relate to a system that pre-screens individuals for OSA. In addition, the present system may relate more particularly to a system that searches health records to pre-screen individuals for undiagnosed medical condition(s), such as OSA, to enable a pathway comprising active case-finding, diagnosis and treatment and a method of operation thereof.

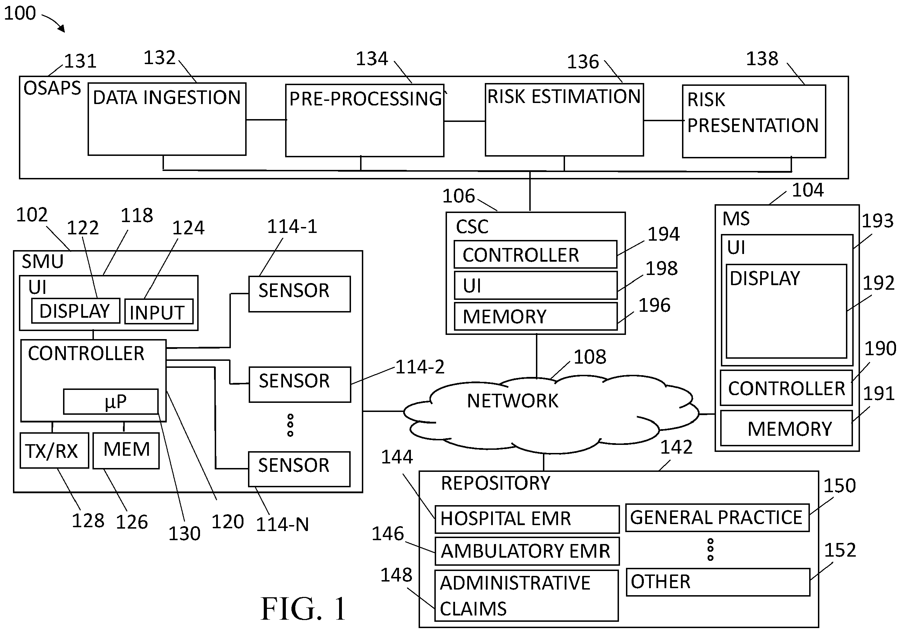

FIG. 1 illustratively shows a schematic block diagram of a portion of a system 100 (hereinafter the system 100) operating in accordance with embodiments of the present system. The system 100 may include one or more of a sleep monitoring unit (SMU) 102, a mobile station (MS) 104, a clinical service center (CSC) 106, a network 108, a repository 142 and an obstructive sleep apnea (OSA) pre-screening stage (OSAPS) 131, one or more of which may communicate with the other via any suitable communication method or methods such as wired, wireless, and/or optical communication methods, such as over the network 108. As discussed, the system 100 including the OSAPS 131 may be adapted to identify undiagnosed medical condition(s) besides OSA with suitable adjustments to system portions and as such, should be understood to be considered in the following discussion and encompassed by the claims that follow.

The network 108 may include any suitable network such as an ad-hoc network, a body area network (BAN), a personal area network (e.g., e.g., Bluetooth™, etc.), a Zigbee network, Wi-Fi, a local area network (LAN), a wide area network (WAN), a telephony network, a cellular network (e.g., 5G, etc.), etc. The SMU 102, the MS 104, the CSC 106, the network 108, the repository 142, and the OSAPS 131 may be controlled by at least one controller which may control the overall operation of the system and may include one or more logic devices such as a microprocessor and/or the like.

The at least one controller may access a memory and may obtain system setting information (SSI) which may include one or more of operating parameters (e.g., scan type, etc.), operating settings, threshold values, warnings, display settings, user information, patient information such as patient identification (ID), patient records, content (e.g., video, audio, etc.), etc. The SSI may be set by the system and/or user and may be updated during use. The at least one controller may generate and render an interface with which the patient and/or clinician may, for example, interact with to, for example, set, reset, select, and/or adjust one or more settings of the SSI and/or enter information (e.g., ID, test type, settings, parameters (e.g., test duration), results, etc.) which may be stored in the SSI in a memory of the system. The test duration may be employed by the system to determine system capacity which may be employed to determine a time bound capacity of the diagnostic pathway such as may be provided by the SMU 102. For example, if test duration is 8 hours, the system may process three tests in a 24 hour period (e.g., 24 hrs/8 hours/test) which may be the capacity of the SMU 102 (although other times are also envisioned). The at least one controller may be a local and/or distributed throughout the system.

The MS 104 may include any suitable mobile station(s) such as a smart-phone (e.g., an I-Phone, a Galaxy phone, etc.), a personal digital assistant (PDA), a smart watch (e.g., an Apple™ Watch™, a Galaxy™ watch, etc.), a smart ring, smart headband (generally, token) and/or the like, and may include a memory 191, at least one controller 190, a user interface (UI) 193, and a transceiver (Tx/Rx) configured to communicate with the network 108.

The UI 193 may provide an interface with which a user (e.g., the patient, the clinician, etc.) may interact, such interfaces may include, inter alia, a touch-screen display 192, an optical sensor such as a camera, a proximity sensor, a speaker, and/or a microphone. However, other user interfaces are also envisioned. The UI 193 may render information, such as content, etc., on a display and/or a speaker for the convenience of the patient or clinician, and may receive information entered by the patient or clinician such as selections, requests, commands, patient or clinician settings, etc., using any suitable input method such as gesture inputs, a touch input, voice input, etc. The memory 191 may store information for use by the system such as the SSI, sleep monitoring information (SMI), system settings, system parameters, operating parameters, and/or combinations thereof, as discussed herein. The patient identification (ID) may be employed to identify the patient and corresponding test results. The MS 104 may transmit information such as operating parameters, instructions, requests, commands (e.g., a synchronization command, etc.), to the SMU 102, and may receive information generated by the SMU 102 such as the SSI, system capacity (or portions thereof), operating parameters, and/or combinations thereof, from the SMU 102 for further processing. The MS 104 may further receive and/or transmit SMI as will be discussed below. Data transfer may occur on periodic and/or non-periodic intervals as may be set forth in the SSI or requested by the system (e.g., the SMU 102, the CSC 106, and/or the MS 104), patient, and/or clinician. For the sake of clarity, only a single MS 104 is discussed unless the context indicates otherwise. However, without limitation other MSs may be employed by the system. For example, a clinician such as a doctor may have an MS which may receive information such as SSI, SMI, etc., from other portions of the system for further review, processing, and/or storage. Accordingly, there may be a plurality of MSs 104 employed by the system 100, one or more of which may communicate with each other and/or to other portions of the system 100, via the network 108 or any suitable communication link.

The CSC 106 may include one or more of a controller 194, a memory 196 and a UI 198. The controller 194 may control the overall operation of the CSC 106 and, as such, may receive information, such as the SSI, SMI and/or other information from the SMU 102 and/or MS 104 for further processing. The controller 194 may control operation of the OSAPS 131 and may transmit queries to the repository 142 and may receive results of the corresponding queries from the repository 142 and provide these to the OSAPS 131, although the OSAPS 131 may also utilize a controller that is separate from the CSC 106. The CSC 106 may further communicate with the SMU 102 and/or MS 104 to, for example, transmit diagnostic results of the processing (e.g., OSA detected, OSA not detected, etc.) to be rendered on a rendering device of the MS 104 for the convenience of the user. The diagnostic results may be stored in the SMI in a memory of the system. The CSC 106 may process data in real time or may obtain data that has been stored in a memory of the system.

One or more controllers of the system 100 may employ machine learning and/or artificial intelligence (AI) engines to process information generated by the system such as the SMI as well as data provided to the system such as historical information and may be operative to detect for example, the presence or absence of obstructive sleep apnea (OSA), the severity of the OSA, etc., may store and/or update results in a memory of the system (e.g., within the SMI), and/or may forward these results to the corresponding patient or clinician for their convenience as for example may be set forth in the SSI. The SMI may be accessed by one or more of the CSC 106, the MS 104 and the SMU 102 of the system for further review, processing, and/or analysis.

The SMU 102 may include one or more of a controller 120, a Tx/Rx 128, a memory 126, sensors 114-1 through 114-N (generally sensors 114, where N is an integer) and a user interface (UI) 118 with which a user, such as the patient or clinician, may interact. The controller 120 may control the overall operation of the SMU 102 and may communicate with one or more of the CSC 106, the MS 104, the network 108, the repository 142 and the OSAPS 131 via the Tx/Rx 128. The controller 120 may include one or more logic devices such as a microprocessor (μP) 130 and may control the overall operation of the SMU 102. It should be appreciated that in some embodiments the controller 120 may include digital and/or analog control circuitry. It is further envisioned that the SMU 102 may be a standalone unit with its own power supply.

The UI 118 may include a display 122 (e.g., a touch-screen display) and a user input interface 124 which may be combined with or separate from each other. The user input interface 124 may include a keyboard, a mouse, a trackball, touch sensitive sensor, or other device, such as a touch-sensitive display, which may be stand alone or part of a system, such as part of a laptop, a personal digital assistant (PDA), a mobile phone (e.g., a smart phone), a smart watch, an e-reader, a monitor, a smart or dumb terminal or other device for communicating with the controller 120 via any operable link such as a wired and/or wireless communication link. The user input interface 124 may be operable for interacting with the controller 120 including enabling interaction within the UI 118 as described herein. Clearly the controller 120, the sensors 114, the user input interface 124, the user interface (UI) 118, the memory 126 and the network 108 may all or partly be a portion of a computer system or other device. The UI 118 may be operative to provide audio/visual feedback as well as graphical user interfaces (GUIs) to the user of the present system and may inform the operator of operating parameters, operating states, etc.

The controller 120 may be operative to render content, such as still or video information, as well as audio information on a rendering device of the system such as on the display 122 and/or speaker of the UI 118. This information may include information related to operating parameters, instructions, feedback, and/or other information related to an operation of the system, or portions thereof, such as SSI or portions thereof, SMI, application data, data generated by the system, operating parameters, etc., and/or combinations thereof. Clinical decision support (CDS) may be employed to analyze data generated by the system and may generate determinations which may be stored in the system and/or may be rendered for the convenience of the patient and/or clinician, for example depending upon settings in the SSI.

The controllers discussed herein, such as the controller 120, may be operable for providing control signals and/or performing operations in response to input signals from the user input device 124 as well as in response to other devices of a network, such as the sensors 114, and executing instructions stored in the memory 126. The $\mu$P 130 may include one or more of a microprocessor, an application-specific and/or general-use integrated circuit(s), a logic device, etc. Further, the $\mu$P 130 may be a dedicated processor for performing in accordance with the present system and/or may be a generalpurpose processor wherein only one of many functions operates for performing in accordance with the present system. The $\mu$P 130 may operate utilizing a program portion, multiple program segments, and/or may be a hardware device utilizing a dedicated or multi-purpose integrated circuit. It is envisioned that one or more portions of the SMU 102, such as the controller 120, may be operationally coupled to the memory 126, the user interface (UI) 118 including a rendering device such as the display 122, the sensors 114, the user input interface 124 and the network 108.

The memory 126 include any suitable memory configured to store information used or generated by the SMU 102, such as operating instructions, the SSI, the SMI, etc. In some embodiments, the memory 126 may store information related to test types such as sensors employed by the test type, instructions for running the test type, the operating flow of the test type, SSI and SMI generated by the test type. The memory 126 may be any type of device for storing application data as well as other data related to the described operation such as data generated by the system, operating parameters, SSI, SMI, etc., and/or combinations thereof. The application data, data generated by the system, operating parameters, SSI, SMI, etc., and/or combinations thereof, may be received by the controller 120 for configuring (e.g., programming) the controller 120 to perform operation acts in accordance with the present system. The controller (e.g., the controller 120) so configured becomes a special purpose machine particularly suited for performing in accordance with embodiments of the present system.

The methods of the present system are particularly suited to be carried out by a computer software program, such program containing modules corresponding to one or more of the individual steps or acts described and/or envisioned by the present system. Such program may of course be embodied in a computer-readable medium, such as an integrated chip, a peripheral device or memory, such as the memory 126 or other memory coupled to the controller 120.

The program and/or program portions contained in the memory 126 may configure the controller 120 to implement the methods, operational acts, and functions disclosed herein. The memories may be distributed, for example between the clients and/or servers, or local, and the controller 120, where additional processors may be provided, may also be distributed or may be singular. The memories may be implemented as electrical, magnetic, optical memory and/or any combination of these or other types of storage devices. Moreover, the term "memory" should be construed broadly enough to encompass any information able to be read from or written to an address in an addressable space accessible by the $\mu$P 130 of the controller 120. With this definition, information accessible through a network such as the network 108 is still within the memory, for instance, because the $\mu$P 130 may retrieve the information from the network 108 for operation in accordance with embodiments of the present system.

The SMU 102 may include any suitable measurement system or systems that monitor the patient's physiological changes over time during a (sleep) study period using one or more test types to determine physiological changes of the patient from which sleep features of the patient may be derived. These test types may have process flows and operations that may be stored in the SSI and may be selected by the SSI, the patient, and/or the clinician as may be desired. In accordance with embodiments of the present system, it is envisioned that the SMU 102 may monitor the patient's physiology over a study period using one or more test types (e.g., each corresponding to a different type of sleep study such as full diagnostic polysomnography, home sleep study, etc.), etc., and form corresponding sensor information which may be processed in accordance with the corresponding test type and thereafter, be stored as test results in the SMI.

The study period may include a time period during which the patient is monitored. This time period may vary depending upon the type of sleep study and information of such may be provided to a controller of the system to determine, for example, a system capacity limit which may be expressed as a rate indicative of a number of patients that can be referred in a given amount of time. Thus, each SMU 102 may have its own capacity limit. These capacity limits may vary in accordance with a test type. Accordingly, the CSC 106 may communicate with the SMUs 102 to determine each of their capacity limits to determine the system capacity limit. Thus, the system may determine the sum of the capacity limits from each SMU 102 to determine an overall capacity limit for use in determining risk as discussed herein. The SMI may include information related to the capacity limit which may be updated in real time by the system to reflect the actual time taken to run each test or test type at the SMU 102. Then, this information may be provided to a controller of the system for further processing and storage in, for example, the SSI. It is also envisioned that the controller of the system may determine the time period (for each SMU) in accordance with a desired capacity limit and may inform each SMU of a time period in which it should perform a sleep study and may store information of such in the SSI. Each SMU may then perform a corresponding sleep study in accordance with its allocated time period.

Each of the test types may employ one of more sensor types to capture biological signals that may then be processed in accordance with their corresponding test type and stored in the SMI for later use. Suitable test types (e.g., types of sleep studies) may include a home-type sleep study, a Polysomnography (PSG) -type study which may monitor one or more bodily functions (e.g., for physiological changes) of the patient, such as brain activity, eye movements, muscle activity, heart rhythm, etc. In accordance with the present system, the information from the sensors is processed to form corresponding sensor information that may be formed into an electrogram, electrooculogram, electromyogram, electrocardiogram, respectively depending upon study type, and may be stored in the SMI for use and/or further processing. The system may apply machine learning algorithms or the like to analyze one or more of the electrogram, electrooculogram, electromyogram, electrocardiogram, and derive, or otherwise determine corresponding SMI. The SMI may then be provided to the CSC 106 for further processing.

It is envisioned that when employing the EEG test type, one or more electrodes, such as may be provided by a Philips™ SmartSleep™ headband, Philips™ NightBalance™ and/or the like, may be employed. In some embodiments, one or more functions of the patient during sleep may be tracked with other modalities either alone, or in combination with, for example the EEG, such as heart rate variability (e.g., measured with ECG, PPG, bed sensors, remote PPG cameras, etc.) or respiratory variability (e.g., measured with surrogate measures of respiratory effort based on bioimpedance, thoracic or abdominal belts, PPG blood volume amplitude variations, bed sensors, radio-frequency (RF)/Doppler radar, infrared cameras, etc.). Thus, the sensors of the system may vary based upon the test type and/or system and/or patient preferences. For example, the sensors 114 may vary in accordance with the (sleep) test type employed to track the one or more bodily functions of the patient during sleep. Similarly, the controller 120 may operate in accordance with the type of sleep test or tests employed to track the one or more signals corresponding to the physiological changes of the patient over the study period and may process these signals in accordance with the test type. In some embodiments, when for example performing a PSG-type test, the sensors may record one or more of eye movement, oxygen and/or carbon dioxide levels in the blood, heart rate, rhythm, breathing rate and rhythm, and airflow through the mouth and nose, and snoring and form corresponding sensor information that may be processed in accordance with the PSG-type test to determine the respiratory status of the patient which may then be stored in the SMI along with the processed signals, etc.

The sensors 114 may be situated at one or more portions of the system and may sense related parameters, form corresponding sensor information, and provide this sensor information to the controller 120 for further processing. For example, the sensors 114 may include sensors which may be specific to a (sleep) test type being performed. The sensors 114 may be distributed throughout the system 100 and/or may be local to the SMU 102. During operation, the controller 120 may receive signals (e.g., analog and/or digital signals) from the sensors 114, amplify (e.g., using one or more amplifiers) and/or condition these signals (e.g., using one or more signal conditioners), convert these signals (e.g., using one or more analog-to-digital (A/D) converters) to digital signals as desired (e.g., at a desired sampling rate (as may be set in the SSI)) to form corresponding information which may be further processed and/or may be stored in the SMI for later use and/or processing to, for example, determine the respiratory status of the patient (e.g., OSA detected, OSA not detected, inconclusive, etc.).

The repository 142 may include one or more healthcare repositories such as, each of which may be considered data sources for medical records such as electronic health records (EHRs), electronic medical records (EMRs), and/or the like and, in this regard, may for example, include one or more data repositories for medical records such as a hospital EMR 144, an ambulatory EMR 146, an administrative claim 148, a general practice EMR 150, another EMR 152, etc. Each of these repositories may have the same or different formats or querying rules for accessing and/or file retrieval such as may be employed by the Centers for Medicare and Medicaid Services (CMS) system. Accordingly, formats and/or querying rules for communicating with (e.g., to query, read files, communicate with, etc.) each of the repositories may be stored in the SSI for later use. To simplify the following discussion, each of these types of electronically stored medical information is referred to herein as an electronic medical record (EMR) though other types of electronically stored health records are understood to be within the scope of the discussion herein and the appended claims. The operation of the OSA processing stage (OSAPS) 131 is described with reference to operations performed on a healthcare repository such as one including CMS insurance claims data which will be the focus of the present description for the sake of clarity. However, without limitation, it should be appreciated that other embodiments of the present system may be operative with other types of healthcare data repositories and/or other patient information without departing from the spirit and scope of the appended claims.

The OSAPS 131 may perform an OSA pre-screening operation as a first step for a pathway to actively find suspected OSA cases in a population (such as a patient cohort) and may include one or more of a data ingestion module 132, a pre-processing module 134, a risk estimation module 136, and a risk presentation module 138, one or more of which may be realized in software and/or hardware, such as specific circuitry, programmable or otherwise, and controlled by a controller of the system such as the controller 194 of the CSC 106.

The data-ingestion module 132 may extract medical status information (MSI) of patients of the patient cohort on demographics, medical history on diagnoses, procedures, symptoms, and/or signs, from one or more selected repositories and may form a corresponding patient cohort dataset (PCD) including the corresponding MSI. The PCD may be stored by the system for further processing. Further, the PCD may be provided to the pre-processing module 134, the risk estimation module 136, and/or the risk presentation module 138 and may be updated by any of these modules. To perform the extraction, the data-ingestion module 132 may be operative to query the selected repositories in accordance with querying rules for the corresponding repository of the selected repositories from the repository 142. The PCD may include the MSI and related information (e.g., ID, etc.) for each patient (e.g., case) of the patient cohort independently.

Accordance with the present system, with regard to the selected repositories, the PCD from the repositories may be linked (e.g., together and/or within the PCD for given repositories) in accordance with various data source entities (DSEs) such as: a patient identifier (ID), an event identifier, a location identifier, coding elements including diseases/diagnoses (e.g., International Classification of Diseases (ICD)-9/10 codes), medical procedures (e.g., Healthcare Common Procedure Coding System (HCPCS)/Current Procedural Terminology (CPT) codes) and prescriptions to provide a complete overview of the PCD. Thus, once the DSE(s) is/are selected (e.g., by the clinician and/or the system), one or more corresponding repositories may be selected accordingly. In some embodiments, the SSI may provide a reference to identify the DSEs and corresponding repositories and vice versa. For example, in the present embodiments, the CMS insurance claims data may be sourced from one or more repositories such as the EMR 144, the ambulatory EMR 146, the general practice EMR 150, and/or the administrative claims 148 data repositories as may be set in the SSI, set by a clinician and/or otherwise selected by the system.

For the sake of simplifying the discussion herein, it is assumed that CMS insurance claims data are ingested to form the PCD, in which case, the MSI may be read from: DENOMINATOR, HOSPICE, INPATIENT, OUTPATIENT, CARRIER, HHA and SNF claim files and revenue center files for the years available or specified (e.g., full year). As appreciated, in the CMS file format/data structure, the DENOMINATOR file is a specific file containing all CMS beneficiaries annually entitled or enrolled even for one day in the calendar year. It is a key data file as it lists all eligible beneficiaries with some essential data about them.

In accordance with the present system, the data-ingestion module 132 may then provide the PCD including the associated MSI to the pre-processing module 134 for further processing. The pre-processing module 134 may then obtain the PCD and may be configured to exclude patient cases/records (hereinafter cases or patient cases) from the patient cohort data of the PCD that are found to be disqualified for further analysis such as: patients without insurance coverage within a specified period, patients who were not alive within a specified period (i.e., patients that died), patients with a confirmed diagnosis of a selected condition (e.g., OSA in the present embodiments) or a related condition, patients who already receive treatment related to the selected condition (e.g., OSA in the present embodiments and which may receive continuous positive airway pressure (CPAP)), patients with an age beyond a relevant age or within or outside a specific age range (e.g., outside of or beyond a threshold age range, for example, the threshold range may be 20 through 70 years old, which may exclude children and those over 70), and patients with a terminal illness, and/or indicated to be in an end-of-life or palliative care stage. The exclusions may be set forth by the exclusion rules (ERS) which may be stored in the SSI and may be set by the system and/or a clinician.

There may be a plurality of ERS sets which may be selected by the clinician and/or system, depending upon the type of data to be ingested (e.g., CMS rules, general practice rules, etc.). For example, the exclusion rules (ERS) may be set to correspond with a selected file system coding, such as CMS claims data and coding. For example, in the following example, it is assumed that the PCD may include CMS claims data using, for example, an ICD-10 coding. The ICD-10-CM (International Classification of Diseases, Tenth Revision, Clinical Modification) is a system used by physicians and other healthcare providers to classify and code all diagnoses, symptoms and procedures recorded in conjunction with hospital care in the United States, which is incorporated herein in its entirety to simplify the discussions herein. Accordingly, acronyms provided correspond with those for CMS claims data and are not provided for the sake of clarity unless the context indicates otherwise. Once again, assuming that the PCD includes information from the CMS claims data using the ICD-10 coding (although other data formats and/or coding schemes are also envisioned), the pre-processing module 134 may identify the following patient cases for the corresponding actions as listed:

Exclude patient cases with an age beyond the specified age and/or age range as may be set by age thresholds, such as greater than or equal to 30 years old and less than or equal to 80 years old (e.g., between 30 and 80 years of age). The process may do this, for example, by locating age in the DENOMINATOR file and excluding cases if the age does not comply with the specified age range thresholds.

Select all beneficiaries/patient cases who have an insurance plan and were alive throughout the years specified. For example, the process may select a continuously insured population with part A & B FFS coverage using the DENOMINATOR file and indicators such as:

"HMO_INDICATOR(x = 1: 12 months) and ENTITLEMENT_BUY_IN_IND(x = 1:12 months)" to identify FFS Part A and B patients. Select only patients that have "HMOINDICATOR == 0" (e.g., 0 = Not a member of HMO) and "ENTITLEMENT_BUY_IN_IND == 3 or C" throughout the 12 indicators/months (3 = Part A and Part B; and C = Part A and Part B (State Buy-in)). Accordingly, in the example provided, this process indicates that all patients selected were insured with FFS Part A and B patients throughout 12 months (full year).

Exclude beneficiaries (patient cases) with a terminal illness, palliative (hospice) care, or with an end-stage renal diagnosis (ESRD) diagnosis: Exclude patient cases who are flagged with ESRD during the period using DENOMINATOR file with "10 = AGED WITHOUT ESRD in

MEDICARE_STATUS_CODE" and "20 = DISABLED WITHOUT ESRD in
MEDICARE_STATUS_CODE." Exclude patient cases who had hospice claims during the period as they are terminally ill and life expectancies of 6 months. Use "DESY_SORT_KEY/*DSYSRTKY"* as beneficiary ID.

Exclude patient cases who have an OSA-related diagnosis already, though patients known with a OSA diagnosis may act as positive cases in a training or validation set (PCD) to estimate /alpha (calibration intercept) and /beta (calibration slope) for a trained model. For example, there are 9 ICD-10 codes which can be used to specify general sleep apnea as a diagnosis to be found in IP claims: "G47.30" (unspecified); "G47.31" (Primary central sleep apnea); "G47.32" (High altitude periodic breathing); "G47.33" (Obstructive sleep apnea (adult) (pediatric)); "G47.34" (Idiopathic sleep related non-obstructive alveolar hypoventilation); G47.35" (Congenital central alveolar hypoventilation syndrome); "G47.36" (Sleep related hypoventilation in conditions classified elsewhere); "G47.37" (Central sleep apnea in conditions classified elsewhere); "G47.39" (Other sleep apnea); "Z99.89" (Used to code dependence of the C-PAP device).

Remove unnecessary columns in the ingested data repository: Select and save only "IP," "OP," "CARRIER," "SNF," "HHA" claims and revenue centers that belong to the base population and relevant columns i.e., "BEN ID," "claim number+date," all ICD codes for diagnosis and procedure). For IP Claims, keep only the columns "DESY_SORT_KEY, CLAIM_NO, CLM_THRU_DT, CLM_DRG_CD," all columns with a prefix named "ICD_DGNS_CD," and all columns with prefix named "ICD_PRCDR_CD."

The process may then update the PCD to reflect the excluded patient cases using any suitable method such as tagging the excluded patient cases in the PCD to indicate that they are excluded. In Accordance with embodiments of the present system, tagged patients may be removed from the list to save search time. In accordance with other embodiments, the process may check whether a person is tagged or not if tagged patients are not excluded. The process may then provide the updated PCD including the still active patient cases (e.g., after the one or more exclusions) to the risk estimation module 136.

In accordance with embodiments of the present system, the ERS may generally define one or more of an age range, an insurance plan, an insured date range, an illness type, and a diagnosis type, and/or combinations thereof, which are to be excluded from (e.g., removed from the dataset) further analysis unless otherwise desired. Accordingly, the PCD may be assessed in accordance with the ERS to determine whether one or more patient cases are to be excluded. This may be done for each patient case in the PCD. Excluded patient cases may be tagged and the PCD may be updated accordingly. The ERS may be defined in the SSI and may be set and/or reset by the system and/or clinician. The updated PCD may then be provided to the risk estimation module 136.

Upon receiving the updated PCD, the risk estimation module 136 may be operative to specify a diagnostic capacity limit (or confirmation target). In accordance with the present system, the diagnostic capacity limit indicates how many or what portion of the population (e.g., the patients in the PCD which were not excluded) should be indicated as positive for the selected condition (e.g., OSA in the present embodiments) and also indicated to be referred to a follow-up diagnostic pathway. In accordance with embodiments of the present system, a diagnostic confirmation target is a threshold value. For example, if an estimated probability (or likelihood) of suspected OSA passes the diagnostic confirmation target (threshold), the case may be considered positive, otherwise negative. Such a threshold may be determined "optimally" by a receiver operating characteristic (ROC) curve analysis and in consultation with clinical experts. A capacity limit is the number of positive cases that one allows (or can handle simultaneously) in the system as a diagnostic follow-up. The capacity limit is based on logistics and resources of being able to reach out and monitor patients. Accordingly, the capacity may be considered as a rate. In addition, when the capacity is limited, one may be stricter by increasing the threshold i.e., only letting patients through who have a high probability of suspected OSA. Likewise, the present system may be laxer with a lower threshold when there is more capacity. It should be appreciated that the diagnostic capacity limit is inherently time bound. In other words, the diagnostic capacity limit (or confirmation target) is expressed as a rate indicating the number of patients that can be referred to follow up diagnostics (e.g., a diagnostic polysomnography or Sleep Study using, for example, the SMU 102) in a given amount of time. In some embodiments, the diagnostic capacity limit may be set by the system and/or clinician and may be stored in the SSI. In some embodiments, it is envisioned that the system may determine the diagnostic capacity limit based upon the sum of the system capacity limit derived from the capacity limit of each of the SMUs 102 in the system 100, which information may be updated in real time and stored in the SSI. For example, there may be 5 SMUs with a capacity of 2 patient cases/per day (e.g., a 24 hour period). Accordingly, the diagnostic capacity limit may be set to $2\times5=10$ patient cases/day (24-hour period).

The risk estimation module 136 may further compute a risk score for the selected condition (e.g., an OSA risk score in the present illustrative embodiments) for each patient case, for example, based on a presence and/or an absence of recognized risk factors in the patient case. Example, patient cases with (insurance) claims may be sought in the specified period that may have corresponding ICD-10 codes or medications referring to common risk factors, symptoms, and/or signs for OSA which may generally be referred to as risk factors as illustrated in Table 1 below.

Table 1

| RISK FACTORS (FOR OSA CONDITIONS) |
| --- |
| male gender as indicated in the DENOMINATOR file or a female with menopausal and other peri- or postmenopausal disorders in women, ICD10 N95 |
| age < 40 (more severe OSA) or age > 60 (more frequent OSA) in the DENOMINATOR file; |
| reported sleep problems ICD-10-CM G47.9 or nocturia ICD-10 F35.1 |
| snoring ICD-10-CM R06.83; |
| respiratory arrest ICD-10-CM R09.2/J96; |
| intermittent oxygen desaturation - SO2 < 90% (e.g., indicative of hypoxemia) ICD-10 R09.02 or bradycardia ICD-10 R00.1 (heart rate (HR) < 40) or cyanosis ICD-10 R23.0; |
| fatigue ICD-10 R53.83; |
| hypertrophy of tonsils ICD-10 J35.1 or other general symptoms and signs ICD-10 R68.89; |
| nicotine dependence, cigarettes, uncomplicated ICD-10 F17.210; |
| alcohol related disorders ICD-10 F10 or opioid use, ICD-10 F11; |
| overweight and/or obesity (e.g., male neck size > 17 inches, waist size > 44 inches) ICD-10 E66; |
| stroke, cerebral infarction ICD-10 163.9; |
| diabetes mellitus ICD-10 E08-E13; |
| history of any heart disease resulting in heart failure, unspecified - ICD-10 I50,9; |
| hypertension ICD-10 110; |
| mood affected disorder - depressive episode ICD-10 F32; |
| post-traumatic stress syndrome, ICD-10 F43.10; |
| sexual dysfunction or impotence, ICD-10 R37 and F52; |
| congenital deformities of skull, face and jaw ICD-10 Q67.4; |
| hypothyroidism, unspecified - ICD-10 E03.9; |
| frailty or age-related physical debility, ICD-10 R54 or age-related osteoporosis, ICD-10 M81.0, or osteosarcopenia ICD-10 M62.84; and |
| medications known to have an adverse side-effect on respiratory effort or are reported as having a respiratory toxicity. These medications are listed under CPT and HCPCS codes and may be obtained from a database (e.g., available at www.ncbi.nlm.nih.gov/pmc/articles/PMC6812077/pdf/pcrj200112.pdf) by the system and stored in the SSI. |

In accordance with the present system, the risk estimation module 136 may then utilize these (22) risk factors (e.g., risk factors, symptoms, and/or signs) and/or others, to determine whether they are present in a patient case, and for each one that is determined to be present, the process may assign a point (e.g., to increase a counter which may initially be set to 0 for each patient case). As readily appreciated, another tallying system may be suitably applied, such as providing a different weight to one or more given risk factors. In any event, thereafter, the process may tally the assigned points (e.g., risk factor points) to determine a total point score which is equal to a total risk score (e.g., an OSA total risk score) for the patient case. Accordingly, the total score for the risk factors (e.g., 22 in the illustrative example), may be referred to as a total risk score and may be determined by calculating the total number of points accumulated for the corresponding patient case. The total risk score in the present embodiments may run from 0 to 22 as shown in Table 1. In yet other embodiments, the process may determine a weighted score calculation for each risk factor (e.g., 22 above for OSA), as various risk factors, symptoms and signs, may contribute to a different extent to the total risk of a suspected condition (e.g., OSA in the present embodiments). These weights may be learned by analyzing training data and may be continuously recalculated and/or updated and stored in the SSI for later use. In this latter case the total risk score may be less than or greater than 22 depending upon the weights and which risk factors are determined to be present.

The risk estimation module 136 may further determine an estimated risk (e.g., as an odds, log odds and/or probability) based upon a logistic function. For example, a logistic function of the total OSA risk score may be used to estimate the predicted probability of suspected OSA (e.g., a risk estimate):

$$\exp(\alpha + (\text{total OSA risk score}) * \beta) / (1 + \exp(\alpha + (\text{total OSA risk score}) * \beta)); \qquad \text{Equation (1)}$$

the predicted odds may be estimated as:

$$\exp(\alpha + (\text{total OSA risk score}) * \beta); \text{ and} \qquad \text{Equation (2)}$$

the predicted log odds may be equal to the linear predictor:

$$\alpha + (\text{total OSA risk score}) * \beta, \qquad \text{Equation (3)}$$

where, $\alpha$ is an intercept that refers to an estimated baseline log odds for suspected OSA when all risk factors are not met (e.g., set to zero) and $\beta$ is a slope . In accordance with embodiments, the slope may be derived using (advanced) regression analysis. Both the intercept $\alpha$ and the slope $\beta$ may be estimated by means of a calibration procedure using (new-unseen) PCD (data) and that has true positive and true negative cases of patient with OSA as outcome or dependent variable. By estimating a logistic regression using the OSA risk score as a covariate on the new-unseen PCD, the coefficients of the logistic model refer to the $\alpha$ and $\beta$. The predicted probability of suspected OSA may then be defined as:

$$\exp(\alpha) / (1 + \exp(\alpha)). \qquad \text{Equation (4)}$$

For example, if the system assumes a baseline probability (BL) of 0.0497 for an OSA diagnosis; a valid intercept $\alpha$ may be given by:

$$\alpha = \ln(\text{BL}) - \ln(1\text{-BL}) = \ln(0.05) - \ln(0.95) \sim -2.95 \qquad \text{Equation (5)}$$

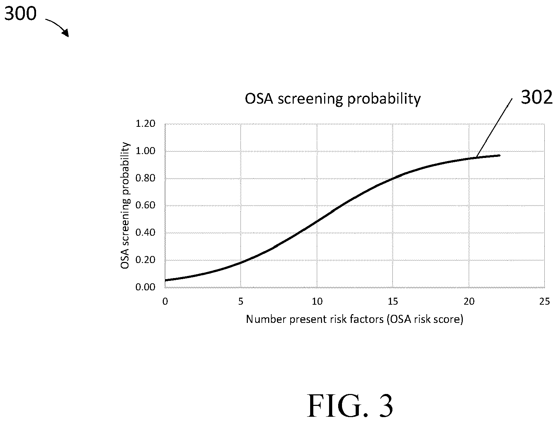

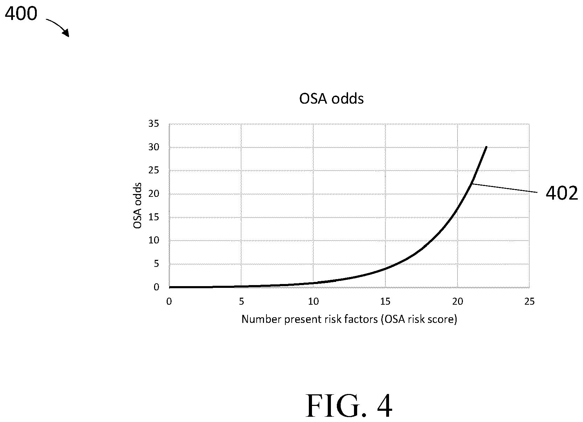

The slope $\beta$ refers to an amount in log odds on the presence of all risk factors, symptoms and/or signs. with $\beta$ = 0.289 as an illustrative acceptable slope. In accordance with embodiments, $\beta$ expresses the spread or extremeness of the probability that someone is suspected of OSA given the present risk factors. This slope is 0.2 /log(2) (there is a 0.2-point increase when the odds double) as a scaling factor enabling the system to use the full scale of 22 points (in the present embodiments, however this value may change depending upon the number of risk factors employed, weighting, etc.) to have a probability running from 0 to 1. These odds and probability computations are illustrated with reference to FIGs. 3 and 4 herein.

FIG. 3 illustratively shows a graph 300 of OSA screening probability as a logistic function of the number of risk factor present in a patient and contained in a OSA risk score in accordance with embodiments of the present system. FIG. 4 illustratively shows a graph 400 of the odds of being susceptible of having OSA as a function of the number of risk factors present in a patient and contained in a OSA risk score in accordance with embodiments of the present system. More particularly, with reference to FIG. 3, a plot 302 of the OSA risk score vs the OSA screening probability is illustrated. In accordance with embodiments of the present system, the plot 302 may be used for the conversion of the total OSA risk score, counting the number of risk factors for suspected OSA, into a predicted (OSA screening) probability for helping in pre-screening for OSA using the illustrated 22 risk factors described above with respect to Table 1. Similarly, with reference to FIG. 4, a plot 402 of the OSA risk score vs. the OSA odds is illustrated. In accordance with embodiments of the present system, the plot 402 may be used for the conversion of the total OSA risk score, counting the number of risk factors for suspected OSA present, into predicted (OSA) odds, for helping in pre-screening for OSA using the illustrated 22 risk factors described above.

Referring back to the intercept $\alpha$ and the slope $\beta$, these values may be adjusted by the system and/or user to avoid overestimation, underestimation, and/or exaggeration of risk of suspected OSA in the patient cohort population. This is especially true if the model is used on an new, unknown cohort or a cohort with specific attributes, such as obese population, morbid population, etc. It is envisioned that the system may adjust the intercept $\alpha$ to correct for underestimation or overestimation of risks. The system may also adjust the slope $\beta$ to correct for any risk under-exaggeration or overexaggeration. For that adjustment, the system may fit the logistic model:

$$Y = \alpha' + \beta' * (\text{total OSA risk score}) \text{ on real data,} \qquad \text{Equation (6)}$$

where, estimated values $\alpha'$ and $\beta'$ are the adjusted intercept and slope, respectively. The system may then provide a stratified random sample of the excluded patient cases (from the PCD) that had a diagnosis already and are treated as positive patient cases in the model fit and the included patient cases without a diagnosis who are treated as the negative patient cases in the model fit. The random sample may be stratified to ensure a correct reflection of the baseline mean probability (prevalence) of an OSA diagnosis in the patient cohort population (e.g., the PCD). For example, an adjustment of $\alpha'$ and $\beta'$ may be performed by the system if sufficient data for model fitting are available. Further, scoring or screening instruments may be validated on a regular basis (e.g., periodically) with data from the cohort, to do a re-calibration.

The risk estimation module 136 may then assign patient cases to a risk category (e.g., low, intermediate, high) or risk level (e.g., integers 1 to 10) as may be set forth in the SSI. For assigning patient cases to a risk category, the system may determine and apply risk thresholds to indicate high risk, intermediate risk, or low risk for OSA. In some embodiments, a direct method may apply thresholds on the predicted probability for OSA. In accordance with embodiments, the thresholds may be predetermined or may be determined from the current PCD. For example, a simple but standard way to find different risk categories from real data (PCD) is by doing a Receiver Operating Characteristics (ROC) analysis in which the OSA risk score is used as a decision variable running from 0 to 22 (for a case wherein there are 22 accessed risks). For every value of the OSA risk score, the sensitivity (true positives or recall) and specificity (true negatives or fall-out) are tabulated or plotted for both patients with OSA and patients without OSA. Two risk score thresholds need to be found - to discern three risk categories. The low risk threshold needs to be found between, for instance, 0 and 5 with good sensitivity and specificity. The high risk threshold needs to be found between 5 and 11 and also should have good sensitivity and specificity. Criteria of what constitutes good sensitivity and specificity dependent on context (e.g., sensitivity + specificity > 1.5).

Then once the thresholds are determined, patient cases may be assigned to a corresponding risk category (e.g., low, intermediate, high), or risk level (e.g., 1 to 10), by matching a corresponding estimated risk with the thresholds for each risk category or risk level. For example, patient cases with a high risk of suspected OSA may have a risk estimate that is determined to be 0.20 or higher. Patient cases with moderate risk may have a risk estimate that is determined to be equal or between 0.10 and 0.20. Patient cases with low risk may have a risk estimate that is below 0.10. High risk cases may be marked as positive cases. However, the chosen threshold values may be changed, but dictate the specificity, sensitivity, and precision, of the pre-screening algorithm. For example, by adjusting one or more of the thresholds, it is possible to maximize specificity at the expense of sensitivity (or vice versa) that is better in "ruling out" or, conversely, "ruling in" OSA. It is further envisioned that in some embodiments, other thresholds may be used, as for example may result from an in-depth analysis of the (updated) PCD, such as may be provided by receiver operating characteristic (ROC) curve analysis, decision curve analysis, and/or the like.

In yet other embodiments, it is envisioned that a method for applying thresholds may employ the log odds for suspected OSA. Accordingly, the system may transform the determined log odds to a negatively scaled OSA score. This score may then be re-scaled, for example, such that every fixed number of point increases of a scale of the OSA (e.g., 50 points of the scale in the present embodiments, although other values are also envisioned) of the scaled OSA score is a doubling of the odds for OSA. This method of scaling is known in the literature as Points to Double the Odds (PDO) scaling. It transforms into points that are deemed to be more meaningful in practice and only needs a linear transformation.

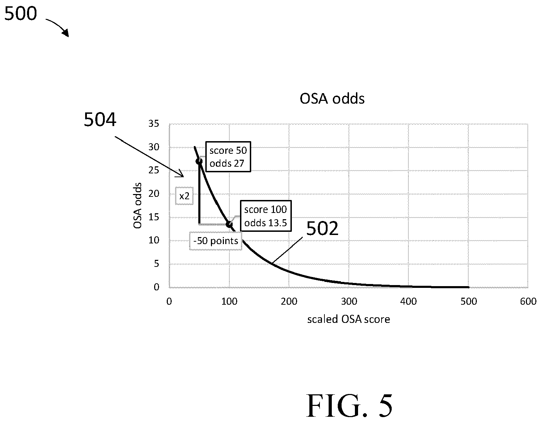

A re-scaling of the OSA score may then be congruent to the computed odds and probabilities of the original OSA risk score. Accordingly, a truncation threshold on the scaled score as multiples of the doubling of the odds. This is illustrated with respect to FIG. 5 which illustratively shows a graph 500 including a re-scaled version of the OSA risk score in accordance with embodiments of the present system. FIG. 5 shows a graph illustrating the odds of being susceptible of having OSA as a function of a scaled OSA score in accordance with embodiments of the present system More particularly, the graph 500 illustrates the OSA risk score, counting the number of risk factors present, transformed such that every 50-point increase (as illustrated by the rise/run 504) in the scaled OSA risk score is indicative of a doubling of the odds. In accordance with embodiments of the present system, thus, assuming that in the graph 500, the scaled OSA score has a start value 500 and 9 truncation thresholds at the scaled OSA score points of 450, 400, 350, 300, 250, 200, 150, 100, 50, <50, then each threshold defines a patient group with a group variation of odds of a factor of 2. Each patient group may be assigned to a risk level (e.g., risk levels 1 to 10). Groups of patient cases may also be rolled up into a secondary risk level hierarchy, for example, of only two or three risk categories (e.g., low, intermediate, high) in accordance with the system settings, as for example may be defined in the SSI.

In accordance with embodiments of the present system, the scaling method may make use of the following formula:

$$\text{Negatively scaled OSA score: } s \text{ (total OSA risk score)} =$$

$$- \delta + \gamma * ( \alpha + \text{(total OSA risk score)} * \beta ), \qquad \text{Equation (7)}$$

where $\gamma$ is a shape factor corresponding to 50-point increase as an odds doubling (= 50 / ln(2) and $\delta$ is an offset and set to 500 - $\gamma$ * ln (1 / exp ($\alpha$) ), thereby

$$\text{Re-scaled OSA score: } r \text{ (total OSA risk score)} =$$

$$(5 - 1) * ( s \text{ (total OSA risk score)} - min ) / (max - min) ) + 1, \qquad \text{Equation (8)}$$

where min and max are respectively the minimum and maximum value of the negatively scaled OSA score s.

Referring back to FIG. 1, the risk estimation module 136 may, for example, determine a follow-up procedure for each patient case based on the determined risk level or risk category for each patient case. Accordingly, the risk estimation module 136 may assign each risk group an OSA follow-up care procedure selected from available procedures such as: (1) no action; (2) OSA screening survey; (3) home analysis; (4) diagnostic polysomnography; and/or other procedures as may be determined by the system, a clinician and/or set in the SSI. In accordance with embodiments of the present system, lowlevel risk groups (such as the lowest level risk group) may be assigned a "not recommended for OSA screening confirmation" (e.g., see, (1) above where the system may exclude a corresponding patient from a diagnostic follow-up for OSA screening). The system may take into account the diagnostic capacity and may refer cases for a diagnostic follow-up starting from the highest risk patient cases (e.g., highest risk patients) and working towards lower risk patients in accordance with the determined risk level (or category) for each patient case in the corresponding risk level (or category) as long as the capacity limit (as discussed above-which is indicative of the diagnostic capacity or the target) has not been exceeded, for a given time period. For example, the system may be set to assign 50 cases a day to follow up procedures such as diagnostic polysomnography if that is the diagnostic capacity or diagnostic target. Thus, in accordance with some embodiments, the system may communicate with the SMUs to determine the cumulative capacity to conduct sleep tests and may employ a feedback loop to determine the system's capacity limit. For example, if the diagnostic capacity limit is 50, the system may recommend 50 cases per day starting with the highest risk patients. The risk estimation module 136 may update each patient case (record) to include information related to the OSA risk score, the estimated risk (as odds, log odds, and/or probability), the predicted probability of suspected OSA, the assigned risk category, and the proposed follow-up procedure(s) and may update the PCD accordingly, store the updated PCD in a memory of the system for later use, and/or may forward the updated PCD to the risk presentation module 138.

The risk presentation module 138, upon receiving the updated PCD from the risk estimation module 136, may then render the determined follow-up procedure(s) (which may include treatment options) for each patient case (in a particular risk level or risk category) and may associate this information with each corresponding patient case and may store this information in a memory of the system for later analysis and/or use. In accordance with embodiments of the present system, various methods exist to present the OSA risk information to the patient, clinician, etc. For example, each patient's case or record may be annotated with a total OSA risk score, and risk level (e.g., high, moderate, low). Further, the risk score may be further annotated with the corresponding risk factors, symptoms and signs for suspected OSA as identified from the data. By opening or printing the patient record, this annotation will help the medical professional to actively find OSA-related complaints during patient consultation. Further, multiple patient cases may be ordered and displayed list-wise on total OSA risk estimate from high to low risk. In this embodiment, the cases at the top of the list are the cases with the highest risk of OSA who require immediate attention from the medical professional. In both presentation cases, the clinician may decide for high risk cases to conduct the next step in the OSA screening and a diagnosis pathway in accordance with embodiments of the present system.

For example, when a clinician accesses the patient case (e.g., such as when viewing the patients' medical records, etc.), the risk presentation module 138 may render, on a rendering device of the system (e.g., display 192, display 122, etc.), one or more of the patient ID, the computed estimated risk, the assigned risk level and/or risk category (e.g., high risk for OSA, etc.), and the determined follow-up procedure for a corresponding patient case as may be determined by the system. Other data from the updated PCD may also be rendered as may be requested by, for example, the patient and/or clinician. The risk presentation module 138 may render a UI such as a graphical user interface (GUI) with which the patient or clinician may interact to view information, update information, schedule the corresponding patient to undergo the determined follow-up procedure or procedures, etc. Thereafter the risk presentation module 138 may further update the corresponding patient cases (record) to reflect any changes that may have been made such as scheduling determined follow-up procedure(s) and may update the PCD accordingly and store this information in a memory of the system (e.g., 196, 191, etc.) for further use.

Embodiments of the present system may provide an OSA processing stage that may employ a pre-screening algorithm to detect patients with suspected but unrecognized OSA by means of a database query or search. By the use of a risk estimation scheme (i.e., a formula), this algorithm may identify patients (represented as records or cases in the database) who have a specific risk (e.g., high, moderate, or low) for suspected OSA by analyzing the recorded data on demographics and clinical history alone for each patient. Patients' cases with high, moderate, or low risk may be indicated as positive, intermediate or negative cases for OSA, respectively. High and intermediate patient cases may subsequently be presented to a clinician as a candidate to be referred to follow-up screening or diagnosis. Follow-up care may include, for example, one or more of: screening with traditional clinical screening tools (e.g., STOP-BANG, and/or the like), nasal flow analyses, a home sleep study, an in-lab sleep study using polysomnography, and/or other treatment/diagnostic options. The treatment options may include one or more of: surgery on the anatomy causing the obstruction (for example, removing the uvula or tonsils), fitting a mandibular repositioning device (MRA), providing continuous positive airway pressure (CPAP), etc. The pre-screening algorithm may be implemented in hardware and/or software and may access patient records in any suitable medical database such as GP (e.g., ambulatory or out-patient) software systems, hospital EMR systems and/or administrative claims data from a health insurer or care provider systems.

Clear advantages of the pre-screening stage provided by embodiments of the present system may include: low cost in its execution; applicable in various clinical domains using GP ambulatory systems, in-patient EMR or administrative claims data from insurance; safe and non-invasive screening; identification of suspected OSA or unknown underlying patients suffering from sleep apnea; increase in specificity (and hence precision) of follow-up procedures; contribution to the overall improvement in yield of finding OSA; and facilitator to initiate an early OSA treatment.

The method may employ common risk factors of OSA including male gender, increasing age (e.g., OSA is more frequent in elderly patients - older than 60 years - and more severe in young patients - younger than 40 years), overweight, large neck size (e.g., > 17 inch for male, > 16 inch for female), enlarged tonsils, craniofacial structure, smoking, drinking, family history, stroke, endocrinopathy and hypertension, comorbidities that are often connected (without knowing the nature) to sleep apnea, diabetes, heart failure, stroke, cancer (e.g., to some extent), obesity, post-traumatic stress syndrome, and depression. Symptoms of OSA to be aware of may include reported sleep problems, daytime sleepiness/fatigue, loud snoring, respiratory arrest or witnessed apneas (e.g., by a third party such as a partner of the patient), restless sleep, abrupt awakenings, nocturia, unrefreshing sleep, dry mouth or sore throat in the morning, nocturnal/early morning chest pain, sexual dysfunction, morning headache, cognitive or concentration problems, low mood, and/or even depression during the day. Signs of OSA which the system may recognize (e.g., may include as part of the risk assessment) may include one or more of oxygen desaturation, bradycardia, and cyanosis.

In accordance with embodiments of the present system, the system may exclude patient cases that disqualify for the OSA risk estimation. Excluded patient cases need to be left out of the analysis or may require specific attention. Exclusion criteria are defined, for example, for patient cases which are not covered by an insurance plan in a specified period; patient cases who are not alive throughout a specified period; patient cases who already have a confirmed diagnosis of OSA or related condition; patient cases who already receive treatment to manage OSA (e.g., CPAP); patient cases who have an age that is beyond a relevant or specific age range; and/or patient cases who are indicated to be in an end-of-life or palliative care stage.

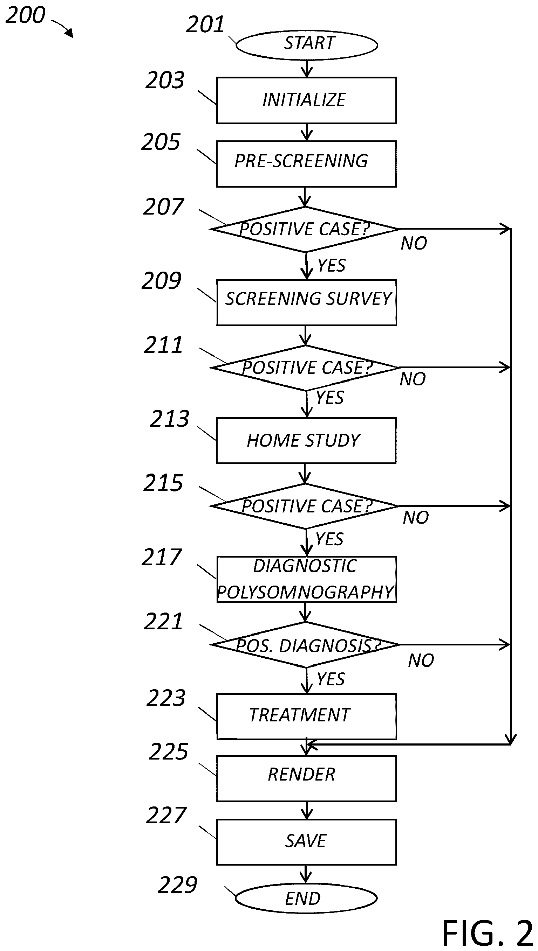

An operational process performed by the system will now be described with reference to FIG. 2 which illustratively shows a functional flow diagram performed by a process 200 in accordance with embodiments of the present system. In accordance with embodiments of the present system, the process 200 provides a pathway to actively find, for example, suspected OSA cases in a population. The process 200 may be performed using one or more processors, computers, controllers, etc., communicating over a network and may obtain information from, and/or store information to, one or more memories which may be local and/or remote from each other. The process 200 may include one of more of the following acts. In accordance with embodiments of the present system, the acts of process 200 may be performed using a controller operating in accordance with embodiments of the present system. Further, one or more of these acts may be combined and/or separated into sub-acts, or performed serially or in parallel, as may be desired. Further, one or more of these acts may be skipped depending upon settings. For the sake of clarity, the process may be described with reference to a single SMU and a single patient case. However, without limitation, it should be understood that the process may employ a plurality of SMUs and a plurality of patient cases each of which may include a separate workflow such as a subworkflow and/or patient cases may be processed within a single work flow.

In operation, the process 200 may start during act 201 and then proceed to act 203. During act 203, the process may initialize by performing an initialization routine. During initialization, the process may load SSI and may set system settings in accordance with the SSI. The process may further obtain machine learning models and/or algorithms that may process data generated by the process 200 in accordance with embodiments of the present system. The SSI may further include information related to test type (e.g., study type), test process flow, test sensor parameters, and/or other settings.

After completing act 203, the process may continue to act 205 during which the process may perform a prescreening operation. For example, the OSA pre-screening stage (e.g., OSAPS 131) may perform an OSA pre-screening operation as discussed herein to ingest data obtained in accordance with a database query or search, form corresponding PCD and thereafter search for risk factors, symptoms, and signs to identify suspected OSA in a patient cohort. Risk estimation may be performed which may include determination of one more risk score(s) which may be further annotated with corresponding risk factors, symptoms and signs for suspected OSA as identified from the data. Thereafter, suspected OSA cases in the patient cohort may be tagged as positive patient cases and the PCD may be updated to reflect this finding. Each of the patient cases that are marked positive (e.g., for risk of OSA) may have an OSA risk assigned thereto such as low, medium, or high. Patient cases with high, moderate, or low risk may be indicated as positive, intermediate, or negative cases for OSA, respectively. For the sake of clarity, positive and intermediate cases will be considered as positive in the present embodiments. After completing act 205, the process may continue to act 207.

During act 207, the process may analyze the PCD to determine whether patient cases from the patient cohort are marked positive (e.g., for OSA). Accordingly, if it is determined that patient cases from the patient cohort are marked positive, the process may continue to act 209. Conversely, if it is determined that no patient cases from the patient cohort are marked positive, the process may continue to act 225.

During act 209, the process may for example perform a screening survey on the patient cases that were marked positive from the patient cohort. Accordingly, a controller of the system may generate and render on a rendering device of the system a user interface with which the clinician and/or patient may interact to provide/answer questions from the screening survey that may be rendered on the rendering device. The answers may be utilized to determine whether the patient has OSA. This screening survey may include, for example, screening with one or more clinical screening tools for screening for sleep apnea such as STOP-BANG™ (SB), Epworth Sleepiness Scale™ (ESS), 4-Variable screening tool™ (4-V) and Berlin Questionnaire™, and/or the like. These clinical screening tools may vary in purpose (e.g., estimating high risk of OSA, detecting daytime sleepiness or insomnia, etc.). Thereafter, patient cases may be marked positive or negative for OSA (e.g., whether it is likely or not that a corresponding patient has OSA) and the PCD may be updated by the system to reflect this accordingly. In addition, a clinician (e.g., a doctor during consultation with a patient) may also screen for OSA symptoms during a patient consult and may add any resultant information to the updated PCD (e.g., addition of risk factor information) for risk analysis as described herein (e.g., by the OSAPS 131). After completing act 209, the process may continue to act 211.

During act 211, the process may determine whether patient cases from the patient cohort (as reflected in the updated PCD that was updated/analyzed during act 209) are determined to be positive. Accordingly, when it is determined that cases from the patient cohort are determined to be positive, the process may continue to act 213. Conversely, when it is determined that no patient cases from the patient cohort are determined to be positive, the process may continue to act 225.

During act 213, a home study (e.g., a sleep study) process may be performed for the patient cases (e.g., for the corresponding patients) that were determined to be positive. Accordingly, the process may interact with sensors (e.g., see, sensors 114, FIG. 1) and/or a controller of a home study system to observe whether the patient exhibits symptoms of OSA. The home study may employ one or more of electrode sensors, nasal flow analysis, and/or other sleep study sensors at home covering one or more nights to determine whether the patient exhibits symptoms of OSA (e.g., whether the patient exhibits physiological symptoms consistent with an OSA diagnosis). For each patient of the patient cohort that undergoes the home study, if it is determined that the corresponding patient exhibits one or more physiological symptoms consistent with an OSA diagnosis, the process may mark (e.g., by the home study) the corresponding patient case positive. Conversely, the home study may mark patient cases negative if it is determined that the corresponding patient does not exhibit OSA symptoms. The process may update the PCD to reflect the findings of the home study.

After completing act 213, the process may continue to act 215 during which it may determine whether patient cases from the patient cohort are marked positive (e.g., for OSA) by the home study. Accordingly, when it is determined that patient cases from the patient cohort are marked positive by the home study, the process may continue to act 217. Conversely, when it is determined that no patient cases from the patient cohort are marked positive by the home study, the process may continue to act 225.

During act 217, the process may perform diagnostic polysomnography (e.g., for example, by the SMU 102 of FIG. 1) on the patient cases that were marked positive during act 209. Accordingly, the process may interact with sensors and/or a controller of a diagnostic polysomnography system to determine whether the patient has detected OSA during the polysomnography testing. If so, the system may mark the patient case for this patient positive in the affirmative (e.g., reflecting a positive diagnosis) by the diagnostic polysomnography. Conversely, during the diagnostic polysomnography, the system may mark a patient case negative (e.g., reflecting a negative diagnosis) if it is determined that the corresponding patient does not have OSA during the polysomnography testing. The process may then update the PCD to reflect the findings of this act. The diagnostic polysomnography may be performed by a sleep study center or the like and may employ full diagnostic polysomnography. This act may confirm and/or otherwise strengthen findings of OSA that may have been determined during previous acts.

After completing act 217, the process may continue to act 221 during which the process may determine whether patient cases from the patient cohort have a positive diagnosis (e.g., from the diagnostic polysomnography act). Accordingly, if it is determined that patient cases from the patient cohort have a positive diagnosis, the process may continue to act 223. Conversely, if it is determined that no patient cases from the patient cohort have a positive diagnosis, the process may continue to act 225.

During act 223, the process may determine one or more treatment plans or options such as health services, CPAP, oral appliances, surgery, weight control, smoking cessation, exercise, limiting alcohol, limiting sedatives, etc., for each patient case from the patient cohort that received a positive diagnosis as a result of the diagnostic polysomnography. The process may update the PCD to reflect the determined treatment plans for each patient case that was determined from the patient cohort to have a positive diagnosis. After completing act 223, the process may continue to act 225.

During act 225 (e.g., following any of acts 207, 211, 215, 221 or 223), results of the process 200 may be rendered. For example, the process may render on a rendering device of the system, an indication (e.g., results/findings of the process 200) of whether a corresponding patient case from the patient cohort has OSA and the determined treatment plans for this patient case. These findings may then be provided to the corresponding patient and/or clinician associated with the patient such as a doctor of the patient, etc. If no patients are found to have OSA, the process may render an indication of such for the convenience of a user and/or clinician. A user interface may be provided to interact with the system.

After completing act 225, the process may continue to act 227, during which the process may update the PCD to reflect the findings and determinations of the process for each patient of the patient cohort. Further, the process may store the updated PCD in a memory of the system for later use and/or processing. After completing act 227, the process may continue to act 229 wherein it may end.

Embodiments of the present system may provide a method that is flexible and may be adjusted or calibrated to a cohort at hand (e.g., a patient cohort). In accordance with embodiments, patient cases with a confirmed diagnosis in the cohort express the mean prior probability or prevalence of OSA in the cohort allowing to correct for over or underestimation of risks for OSA in a given population. It is also envisioned that the method may be adjusted to correct for over- or under-exaggeration of OSA risk in a population, such as cases with a diagnosis who would receive a too low (or too high) risk estimate and cases without a diagnosis who would receive a too high (or too low) risk estimate.

Embodiments of the present system may be integrated into various sleep apnea solutions and sleep monitoring solutions. Next to the home monitoring setting, the solution may also be used to screen for sleep apnea in hospitals, institutions, nursing homes, and/or other care settings. As operations may be automated, a clinician may not be necessary for performing sleep analysis using embodiments of the present system, rather a patient may employ sensors coupled to a controller to perform a home sleep study or the like. This can enhance user convenience and reduce costs.

Further variations of the present system would readily occur to a person of ordinary skill in the art and are encompassed by the following claims.

Finally, the above discussion is intended to be merely illustrative of the present system and should not be construed as limiting the appended claims to any particular embodiment or group of embodiments. Thus, while the present system has been described with reference to exemplary embodiments, it should also be appreciated that numerous modifications and alternative embodiments may be devised by those having ordinary skill in the art including using features that are described with regard to a given embodiment with other envisioned embodiments without departing from the broader and intended spirit and scope of the present system as set forth in the claims that follow. In addition, any section headings included herein are intended to facilitate a review but are not intended to limit the scope of the present system. In addition, the specification and drawings are to be regarded in an illustrative manner and are not intended to limit the scope of the appended claims.

In interpreting the appended claims, it should be understood that:

    a) the word "comprising" does not exclude the presence of other elements or acts than those listed in a given claim;
    b) the word "a" or "an" preceding an element does not exclude the presence of a plurality of such elements;
    c) any reference signs in the claims do not limit their scope;
    d) several "means" may be represented by the same item or hardware or software implemented structure or function;
    e) any of the disclosed elements may be comprised of hardware portions (e.g., including discrete and integrated electronic circuitry), software portions (e.g., computer programming), and any combination thereof;
    f) hardware portions may be comprised of one or both of analog and digital portions;

g) any of the disclosed devices, features and/or portions thereof may be combined together or separated into further portions unless specifically stated otherwise;

h) no specific sequence of acts or steps is intended to be required unless specifically indicated; and

i) the term "plurality of" an element includes two or more of the claimed element, and does not imply any particular range of number of elements; that is, a plurality of elements may be as few as two elements, and may include an immeasurable number of elements.

**Claims**

1. A screening system (100), comprising:

   at least one controller (120, 190, 194) configured to:
   query a plurality of selected data sources (142, 144, 146, 148, 150, 152) for medical records to extract medical status information (MSI) of patients in a patient cohort;
   form patient cohort data (PCD) comprising patient case records, each of the patient case records comprising the extracted MSI of the patients in the patient cohort;
   exclude patient case records from the PCD that are determined to be disqualified according to exclusion rules (ERS), and update the PCD accordingly;
   determine risk factors that are present for each patient case record of the updated PCD in accordance with a comparison of the MSI for corresponding patient case records with risk factors for obstructive sleep apnea (OSA);
   determine a total risk score for each of the corresponding patient case records in accordance with a total of the determined risk factors that are determined to be present for the corresponding patient case records;
   determine a predicted probability of suspected OSA based upon a logistic function of the determined total risk score for each of the corresponding patient case records;
   assign each of the corresponding patient case records to a risk category in accordance with risk thresholds applied to the predicted probability of suspected OSA; and
   determine a follow-up procedure for each of the corresponding patient case records in accordance with the assigned risk category for the corresponding patient case records.

2. The screening system of claim 1, wherein the at least one controller is further configured to render the determined follow-up procedure on a rendering device (112, 192, 198) of the system under the control of the at least one controller.

3. The screening system of claim 2, wherein the at least one controller is further configured to render one or more of the total risk score, the predicted probability of suspected OSA, and the determined risk category on the rendering device.

4. The screening system of claim 1, wherein the at least one controller is further configured to determine an estimated risk as at least one of odds, log odds, and probability based upon a logistic function of the determined total risk score for each of the corresponding patient case records.

5. The screening system of claim 1, wherein the at least one controller is further configured to determine a system capacity limit.

6. The screening system of claim 5, wherein the at least one controller is further configured to communicate with at least one sleep monitoring unit (SMU) (102) and to determine a cumulative capacity of the at least one SMU to conduct sleep tests and base the system capacity limit in accordance with a sum of the determined cumulative capacity.

7. The screening system of claim 1, wherein the at least one controller is further configured to render a diagnostic follow-up based on the assigned category risk.

8. A screening system (100) for an undiagnosed medical condition, comprising:
   at least one controller (120, 190, 194) which is configured to:

   query a plurality of selected data sources (142, 144, 146, 148, 150, 152) for medical records to extract medical status information (MSI) of patients in a patient cohort;
   form patient cohort data (PCD) comprising patient case records, each of the patient case records comprising the extracted MSI of the patients in the patient cohort;

exclude patient case records from the PCD that are determined to be disqualified according to exclusion rules (ERS), and update the PCD accordingly;

determine risk factors that are present for each patient case record of the updated PCD in accordance with a comparison of the MSI for the corresponding patient case records with risk factors associated with the undiagnosed medical condition;

determine a total risk score for each of the corresponding patient case records in accordance with a total of the determined risk factors that are determined to be present for the corresponding patient case records;

determine a predicted probability of the undiagnosed medical condition based upon a logistic function of the determined total risk score for each of the corresponding patient case records; and

assign each patient case record to a risk category or risk level in accordance with risk thresholds applied to the predicted probability of the undiagnosed medical condition.

9. The screening system of claim 8, wherein the at least one controller is further configured to determine a follow-up procedure for each patient case record in accordance with the assigned risk category or risk level for the corresponding patient case records.

10. The screening system of claim 9, wherein the at least one controller is further configured to render the determined follow-up procedure on a rendering device (112, 192, 198).

11. A method for screening for a given undiagnosed medical condition in a patient cohort, the method comprising acts of:

querying a plurality of selected data sources (142, 144, 146, 148, 150, 152) for medical records to extract medical status information (MSI) of patients in the patient cohort;

forming patient cohort data (PCD) comprising patient case records, each of the patient case records comprising the extracted MSI of the patients in the patient cohort;

excluding patient case records from the PCD that are determined to be disqualified according to exclusion rules (ERS), and updating the PCD accordingly;

determining risk factors that are present for each patient case record of the updated PCD in accordance with a comparison of the MSI for the corresponding patient case records with risk factors associated with the undiagnosed medical condition;

determine a total risk score for each of the corresponding patient case records in accordance with a total of the determined risk factors that are determined to be present for the corresponding patient case records;

determining a predicted probability of the undiagnosed medical condition based upon a logistic function of the determined total risk score for each of the corresponding patient case records; and

assigning each patient case record to a risk category or risk level in accordance with risk thresholds applied to the predicted probability of the undiagnosed medical condition.

12. The method of claim 11, comprising an act of determining a follow-up procedure for each patient case record in accordance with the assigned risk category or risk level for the corresponding patient case records.

13. The method of claim 12, comprising an act of rendering the determined follow-up procedure on a rendering device (112, 192, 198).

14. The method of claim 11, comprising acts of:

annotating the updated PCD with one or more of the total risk score, the estimated risk predicted probability of the undiagnosed medical condition, and the determined risk category or risk level; and

storing the annotated PCD on a storage device (126, 191, 196).

15. The method of claim 11, comprising acts of:

determining a cumulative capacity of a plurality of monitoring units (102) to conduct tests; and

determining a follow-up procedure for each patient case record in accordance with the assigned risk category or risk level for the corresponding patient case records and in accordance with the determined cumulative capacity of the plurality of monitoring units (102).

FIG. 1

**200**

**201** → START

**203** INITIALIZE

**205** PRE-SCREENING

**207** POSITIVE CASE? — NO

YES

**209** SCREENING SURVEY

**211** POSITIVE CASE? — NO

YES

**213** HOME STUDY

**215** POSITIVE CASE? — NO

YES

**217** DIAGNOSTIC POLYSOMNOGRAPHY

**221** POS. DIAGNOSIS? — NO

YES

**223** TREATMENT

**225** RENDER

**227** SAVE

**229** END

FIG. 2

300

OSA screening probability 302

FIG. 3

FIG. 4

500

504

OSA odds

FIG. 5

Europäisches
Patentamt

European
Patent Office

Office européen
des brevets

# EUROPEAN SEARCH REPORT

Application Number

EP 23 15 4488

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/273579 A1 (WRIGHT DAVID BRUCE [US] ET AL) 27 August 2020 (2020-08-27) * paragraphs [0024], [0114], [0138]; claim 12 * | 1-15 | INV. G16H10/60 G16H20/70 |
| A | US 2021/193320 A1 (SHUKLA OODAYE [US] ET AL) 24 June 2021 (2021-06-24) * paragraphs [0005], [0097] * | 8-15 | |
| A | US 2021/158894 A1 (LI JINGJING [US] ET AL) 27 May 2021 (2021-05-27) * the whole document * | 1-15 | |
| A | CN 113 875 187 A (RESMED INC) 31 December 2021 (2021-12-31) * the whole document * | 1-15 | |
| A | US 2016/067220 A1 (GARCIA DA ROCHA MARCELO [US] ET AL) 10 March 2016 (2016-03-10) * the whole document * | 1-15 | TECHNICAL FIELDS SEARCHED (IPC) |
| A | WO 2020/139845 A1 (RESMED INC [US]) 2 July 2020 (2020-07-02) * paragraph [363ff] * | 1-15 | G16H |

The present search report has been drawn up for all claims

| Place of search | Date of completion of the search | Examiner |
|---|---|---|
| Munich | 2 August 2023 | Laub, Christoph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 386 768 A1**

## ANNEX TO THE EUROPEAN SEARCH REPORT
### ON EUROPEAN PATENT APPLICATION NO.

EP 23 15 4488

This annex lists the patent family members relating to the patent documents cited in the above-mentioned European search report.
The members are as contained in the European Patent Office EDP file on
The European Patent Office is in no way liable for these particulars which are merely given for the purpose of information.

02-08-2023

| Patent document cited in search report | | | Publication date | Patent family member(s) | | Publication date |
|---|---|---|---|---|---|---|
| US 2020273579 | A1 | | 27-08-2020 | AU 2018355173 A1 | | 28-05-2020 |
| | | | | CA 3077703 A1 | | 02-05-2019 |
| | | | | CO 2020006178 A2 | | 19-06-2020 |
| | | | | EP 3701543 A1 | | 02-09-2020 |
| | | | | US 2019122769 A1 | | 25-04-2019 |
| | | | | US 2020273579 A1 | | 27-08-2020 |
| | | | | WO 2019083897 A1 | | 02-05-2019 |
| US 2021193320 | A1 | | 24-06-2021 | US 11145419 B1 | | 12-10-2021 |
| | | | | US 11250950 B1 | | 15-02-2022 |
| | | | | US 11270797 B1 | | 08-03-2022 |
| | | | | US 2021193320 A1 | | 24-06-2021 |
| | | | | US 2021327594 A1 | | 21-10-2021 |
| US 2021158894 | A1 | | 27-05-2021 | US 2021158894 A1 | | 27-05-2021 |
| | | | | WO 2019139950 A1 | | 18-07-2019 |
| CN 113875187 | A | | 31-12-2021 | AU 2019417727 A1 | | 22-07-2021 |
| | | | | CN 113875187 A | | 31-12-2021 |
| | | | | EP 3903448 A1 | | 03-11-2021 |
| | | | | JP 2022515534 A | | 18-02-2022 |
| | | | | KR 20210113622 A | | 16-09-2021 |
| | | | | US 11508484 B1 | | 22-11-2022 |
| | | | | US 2022076822 A1 | | 10-03-2022 |
| | | | | WO 2020139845 A1 | | 02-07-2020 |
| US 2016067220 | A1 | | 10-03-2016 | AU 2012321246 A1 | | 01-05-2014 |
| | | | | AU 2017206211 A1 | | 03-08-2017 |
| | | | | AU 2019203564 A1 | | 13-06-2019 |
| | | | | BR 112014008983 A2 | | 02-05-2017 |
| | | | | CA 2851780 A1 | | 18-04-2013 |
| | | | | CA 2978258 A1 | | 18-04-2013 |
| | | | | CN 103957906 A | | 30-07-2014 |
| | | | | CN 109700806 A | | 03-05-2019 |
| | | | | CY 1121041 T1 | | 11-12-2019 |
| | | | | DK 2766014 T3 | | 24-09-2018 |
| | | | | EP 2766014 A1 | | 20-08-2014 |
| | | | | EP 3446691 A1 | | 27-02-2019 |
| | | | | ES 2685814 T3 | | 11-10-2018 |
| | | | | HK 1200333 A1 | | 07-08-2015 |
| | | | | HR P20181378 T1 | | 19-10-2018 |
| | | | | HU E039671 T2 | | 28-01-2019 |
| | | | | JP 6270727 B2 | | 31-01-2018 |
| | | | | JP 6640164 B2 | | 05-02-2020 |
| | | | | JP 2014528474 A | | 27-10-2014 |
| | | | | JP 2018027971 A | | 22-02-2018 |

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 23 15 4488

This annex lists the patent family members relating to the patent documents cited in the above-mentioned European search report.
The members are as contained in the European Patent Office EDP file on
The European Patent Office is in no way liable for these particulars which are merely given for the purpose of information.

02-08-2023

| Patent document cited in search report | Publication date | Patent family member(s) | | Publication date |
|---|---|---|---|---|
| | | JP | 2020073529 A | 14-05-2020 |
| | | JP | 2022008828 A | 14-01-2022 |
| | | KR | 20140094539 A | 30-07-2014 |
| | | KR | 20190029775 A | 20-03-2019 |
| | | KR | 20200019791 A | 24-02-2020 |
| | | LT | 2766014 T | 27-08-2018 |
| | | MX | 362626 B | 28-01-2019 |
| | | PL | 2766014 T3 | 28-02-2019 |
| | | PT | 2766014 T | 11-10-2018 |
| | | SI | 2766014 T1 | 30-11-2018 |
| | | US | 8324260 B1 | 04-12-2012 |
| | | US | 2013096170 A1 | 18-04-2013 |
| | | US | 2013096171 A1 | 18-04-2013 |
| | | US | 2013096172 A1 | 18-04-2013 |
| | | US | 2014005243 A1 | 02-01-2014 |
| | | US | 2016067220 A1 | 10-03-2016 |
| | | US | 2019269657 A1 | 05-09-2019 |
| | | US | 2021137891 A1 | 13-05-2021 |
| | | WO | 2013055528 A1 | 18-04-2013 |
| WO 2020139845 A1 | 02-07-2020 | AU | 2019417727 A1 | 22-07-2021 |
| | | CN | 113875187 A | 31-12-2021 |
| | | EP | 3903448 A1 | 03-11-2021 |
| | | JP | 2022515534 A | 18-02-2022 |
| | | KR | 20210113622 A | 16-09-2021 |
| | | US | 11508484 B1 | 22-11-2022 |
| | | US | 2022076822 A1 | 10-03-2022 |
| | | WO | 2020139845 A1 | 02-07-2020 |

EPO FORM P0459

For more details about this annex : see Official Journal of the European Patent Office, No. 12/82

page 2 of 2

**REFERENCES CITED IN THE DESCRIPTION**

**Non-patent literature cited in the description**

- **ADAM B. BENJAFIELD.** Estimation of the Global Prevalence and Burden of Obstructive Sleep Apnea: A Literature-Based Analysis. *Lancet*